# FASCICULE DE BREVET EUROPEEN

(11) **EP 3 345 646 B1**
(45) Date de publication et mention de la délivrance du brevet: **29.05.2019**
(21) Numéro de dépôt: 17196870.4
(22) Date de dépôt: 17.10.2017
(51) Int. Cl.: A61M 16/16, A61M 16/10, H05B 3/00

(54) **HUMIDIFICATEUR POUR APPAREIL D'ASSISTANCE RESPIRATOIRE**
BEFEUCHTER FÜR BEATMUNGSGERÄT
HUMIDIFIER FOR RESPIRATORY APPARATUS

(30) Priorité: 05.01.2017 FR 1750098
(43) Date de publication de la demande: 11.07.2018
(73) Titulaire: Air Liquide Medical Systems, 92160 Antony (FR)
(72) Inventeur: LEBATTEUR, Nicolas, 91220 BRETIGNY SUR ORGE (FR); LIBARDI, Mickael, 94270 LE KREMLIN-BICETRE (FR); MOVSCHIN, Antoine, 75014 PARIS (FR)
(74) Mandataire: Pittis, Olivier

(56) Documents cités:
- EP-A1- 2 848 277
- WO-A1-2008/148154
- WO-A1-2010/140903
- WO-A1-2012/171072
- US-A1- 2009 107 980
- US-A1- 2012 248 636

## Description

L'invention concerne un humidificateur de gaz pour appareil d'assistance respiratoire, c'est-à-dire pour ventilateur médical, servant au traitement de patients à domicile, notamment de patient souffrant d'une maladie respiratoire de type apnée du sommeil, syndrome d'obésité hypoventilation, broncho-pneumopathie chronique obstructive (BPCO) ou syndrome de détresse respiratoire aiguë (SDRA), ainsi qu'un tel ventilateur intégrant un tel humidificateur de gaz, en particulier d'air.

Certains appareils d'assistance respiratoire, encore appelés ventilateurs, utilisables pour le traitement de patients à domicile, intègrent un humidificateur chauffant servant à humidifier le gaz fourni aux patients, lequel humidificateur chauffant comprend généralement un réservoir et un boitier dans lequel le réservoir vient se loger.

Le réservoir est destiné à contenir un volume d'eau et est conçu pour permettre une circulation d'un débit d'air entre un orifice d'entrée et un orifice de sortie. Le fond du réservoir comporte une plaque métallique chauffante servant à transmettre de la chaleur à l'eau, c'est-à-dire à chauffer l'eau pour la vaporiser et charger ainsi le flux d'air en vapeur d'eau de manière à l'humidifier.

Le boitier est conçu pour recevoir et loger le réservoir. Le fond du boitier comporte une structure chauffante comprenant un élément chauffant et une plaque métallique permettant de transmettre la chaleur produite par l'élément chauffant à la plaque métallique chauffante du réservoir.

Le contrôle de l'humidification du gaz, typiquement de l'air, se fait via l'élément chauffant du boitier dont on pilote la chauffe en fonction, entres autres, de données de température. En général, un tel élément chauffant comprend notamment un élément résistif, typiquement une piste résistive qui dissipe de la chaleur lorsqu'elle est parcourue par un courant électrique, et un ou plusieurs capteur(s) de température permettant de mesurer la température de la plaque métallique et/ou d'une zone proche de la piste résistive qui n'est pas en contact avec la plaque métallique. Les éléments chauffants sont généralement pourvus d'un composant jouant le rôle de fusible thermique, qui coupe le courant circulant dans la piste résistive lorsque la température dépasse un seuil donné.

Lorsqu'une sonde de température ne touche pas la plaque métallique, la valeur qu'elle mesure est représentative de la température de l'élément chauffant, c'est-à-dire la température qui découle de la chauffe générée par la piste résistive. En revanche, ce n'est pas le cas lorsque la sonde de température est en contact avec la plaque métallique car, du fait des propriétés de conductivité thermique de la plaque, sa température résulte à la fois de la chaleur produite par l'élément chauffant et des éléments qui se trouvent de l'autre côté de la plaque. En particulier, lorsque le réservoir est rempli d'eau froide et que sa plaque métallique est en contact avec la plaque métallique sur laquelle est collé l'élément chauffant, la température mesurée par la sonde de température en contact avec la plaque sera fortement diminuée par l'importante capacité thermique que représente le volume d'eau contenu dans le réservoir.

Le document WO2012/171072 décrit un humidificateur pour appareil d'assistance respiratoire comprenant un boitier, un réservoir d'eau à chauffer, une plaque métallique, un élément chauffant et un capteur thermique. US-A-2009/0107980 et US-A-2012/0248636 enseignent des humidificateurs similaires.

Au vu de cela, le problème qui se pose est dès lors d'améliorer le pilotage de l'humidificateur pour qu'il réponde aux exigences de performance et de sécurité.

La solution selon la présente invention concerne un humidificateur pour appareil d'assistance respiratoire comprenant un boitier adapté pour recevoir, c'est-à-dire pour loger, un réservoir d'eau à chauffer, ledit boitier comprenant une structure chauffante agencée dans le fond dudit boitier, ladite structure chauffante comprenant une première plaque métallique et au moins un élément chauffant agencé de manière à transmettre la chaleur produite par ledit au moins un élément chauffant à ladite première plaque métallique, et une première sonde de température en contact direct avec la première plaque métallique, caractérisé en ce que le boitier comprend en outre une seconde sonde de température en contact ou quasi-contact de l'élément chauffant et isolée thermiquement de la première plaque métallique.

En d'autres termes, la présente invention propose de mettre en oeuvre conjointement deux sondes de température dans la structure chauffante de manière à pouvoir améliorer le pilotage de l'humidificateur, à savoir :
- une première sonde de température en contact direct de la première plaque métallique chauffante de sorte de pouvoir, en fonctionnement normal, disposer d'une mesure représentative de la température de l'eau contenue dans le bac du réservoir, et
- une seconde sonde de température en contact ou localisée dans une zone à proximité immédiate de l'élément chauffant, en particulier une piste résistive, de la structure chauffante et isolé thermiquement de la première plaque métallique, c'est-à-dire en contact ou quasi-contact avec l'élément chauffant de sorte de disposer d'une mesure représentative de la température délivrée par ledit élément chauffant, telle une piste résistive.

En effet, pour les besoins de performance, il convient de disposer d'une mesure qui soit représentative de la température de l'eau du bac du réservoir. Cette mesure de température de l'eau peut être estimée à l'aide d'une première sonde de température en contact avec la plaque métallique chauffante. Plus précisément, la température de l'eau n'est pas directement mesurée par la sonde mais la sonde donne une valeur représentative de la température de l'eau, au fond du réservoir.

Par ailleurs, pour les besoins de sécurité, on a besoin d'une mesure qui permette de vérifier, d'une part, que les parties accessibles à l'utilisateur, en particulier un patient, notamment les plaques métalliques, sont maintenues dans une plage de température qui ne présente pas de risque de blessure par brûlure et, d'autre part, que les composants qui sont portés à des températures élevées ne risquent pas de se dégrader sous l'effet de la chaleur. Cette mesure de température de l'élément chauffant est délivrée par la seconde sonde de température qui n'est pas en contact avec la première plaque métallique chauffante mais qui mesure la température de l'élément chauffant, en étant en contact ou quasi-contact avec celui-ci. Ainsi, la seconde sonde de température mesure la température à proximité immédiate de cet élément chauffant, telle une piste résistive. En d'autres termes, la deuxième sonde permet de mesurer la température des zones les plus chaudes de la structure chauffante.

La présente invention propose d'utiliser ces mesures de température opérées par les première et seconde sondes de température pour réaliser un pilotage, par exemple via un algorithme dédié, du courant traversant l'élément chauffant, en particulier une piste résistive, de façon à garantir l'intégrité des différents composants de la structure chauffante face à la température.

Par exemple, la couche adhésive permettant le contact entre l'élément chauffant et la plaque métallique peut être sensible aux températures élevées et le fait de contrôler la température intrinsèque de l'élément chauffant permet d'éviter la dégradation de cette couche.

De même, tout ou partie de ces mesures de température peut aussi être utilisé pour assurer une fonction de fusible thermique et donc une coupure de l'alimentation électrique de l'élément chauffant en cas de détection d'une température excessive, c'est-à-dire supérieure à un seuil de sécurité prédéfini.

Selon le cas, l'humidificateur de l'invention peut comprendre l'une ou plusieurs des caractéristiques techniques suivantes :
- la seconde sonde de température est en quasi-contact de l'élément chauffant en étant en contact avec une zone située autour et à proximité immédiate de l'élément chauffant.
- la seconde sonde de température est en contact avec une zone située à une distance D comprise entre environ 0,1 et 1 mm de l'élément chauffant.
- l'élément chauffant comprend au moins un élément résistif dissipant, i.e. conçu pour dissiper, de la chaleur lorsqu'il est parcouru par un courant électrique.
- l'élément chauffant comprend au moins un élément résistif de forme longiligne, notamment filaire, par exemple un circuit sérigraphié en cuivre, en aluminium ou en un alliage à base de cuivre ou d'aluminium.
- l'élément chauffant comprend un ou plusieurs éléments résistifs.
- l'élément chauffant est relié électriquement à une source de courant électrique.
- l'élément chauffant est relié électriquement au secteur électrique via un cordon d'alimentation munie d'une prise ou d'un connecteur adapté ou à une ou plusieurs batteries électriques.
- au moins une couche adhésive permet de solidariser l'élément chauffant à la plaque métallique de la structure chauffante. Par exemple, la (ou les) couche adhésive est composée de colle.
- ladite au moins une couche adhésive est prise en sandwich entre la première plaque métallique et une première couche isolante électriquement.
- au moins une première couche isolante électriquement est prise en sandwich entre ladite au moins une couche adhésive et ledit élément résistif
- l'élément résistif est pris en sandwich entre au moins une première couche isolante électriquement et au moins une seconde couche isolante électriquement et thermiquement.
- la ou chaque couche isolante électriquement comprend ou est formée de silicone, de polyimide ou analogue, en particulier d'un film en polyimide.
- les première et seconde sondes de température sont reliées électriquement à des moyens de pilotage.
- les moyens de pilotage comprennent une carte électronique.
- les moyens de pilotage recueillent et traitent les signaux de mesure délivrés par les première et seconde sondes de température.
- les première et seconde sondes de température sont par exemple des sondes de type thermistance, thermistance à coefficient de température négatif ou thermistance à coefficient de température positif.
- il comporte un réservoir à eau formé d'un bac comprenant un fond de bac incorporant une deuxième plaque métallique, ladite deuxième plaque métallique du réservoir venant en contact direct avec la première plaque métallique de la structure chauffante du boitier lorsque le réservoir est logé dans le boitier de manière à ce que la chaleur de chauffage passe de ladite première plaque métallique de la structure chauffante du boitier à la deuxième plaque métallique du bac de réservoir, puis à l'eau contenue dans le bac.
- la première sonde de température peut être plaquée contre la première plaque métallique de la structure chauffante au moyen d'un patch supplémentaire isolant électriquement. De préférence, le patch isolant électriquement est formé de silicone.

L'invention porte en outre sur un appareil d'assistance respiratoire comprenant un humidificateur selon l'invention, en particulier tel que décrit dans la présente description et illustré sur les Figures, l'appareil d'assistance respiratoire connecté à l'humidificateur selon l'invention forment un ensemble de ventilation utilisable pour mettre en oeuvre un traitement de patients à domicile, notamment de patient souffrant d'une maladie respiratoire de type apnée du sommeil, syndrome d'obésité hypoventilation, broncho-pneumopathie chronique obstructive (BPCO) ou syndrome de détresse respiratoire aiguë (SDRA).

En d'autres termes, l'invention porte également sur un ensemble de ventilation comprenant un humidificateur selon l'invention raccordé fluidiquement à la sortie de gaz d'un appareil d'assistance respiratoire, tel un ventilateur médical, de sorte d'assurer une humidification du gaz délivré par le ventilateur, tel de l'air.

Dans un tel ensemble, l'humidificateur est raccordé fluidiquement à une interface patient, tel un masque, des canules nasales ou analogue, via un conduit de gaz, tel un tuyau flexible, de manière à pouvoir convoyer le gaz humidifié depuis la sortie de l'humidificateur jusqu'aux voies aériennes du patient.

L'invention va maintenant être mieux comprise grâce à la description détaillée suivante, faite à titre illustratif mais non limitatif, en référence aux figures annexées parmi lesquelles :
- la Figure 1 représente une vue schématique partielle d'un humidificateur de gaz pour appareil d'assistance respiratoire selon l'invention,
- la Figure 2 schématise une structure chauffante (vue en coupe transversale) de l'humidificateur de la Figure 1,
- la Figure 3 schématise une structure chauffante (vue de dessus) de l'humidificateur de la Figure 1,
- les Figures 4 et 5 représentent des évolutions de température lorsque le bac du réservoir de l'humidificateur de la Figure 1, est plein d'eau ou vide, respectivement, et
- la Figure 6 représente un mode de réalisation d'un humidificateur de gaz selon l'invention assemblé à un appareil d'assistance respiratoire.

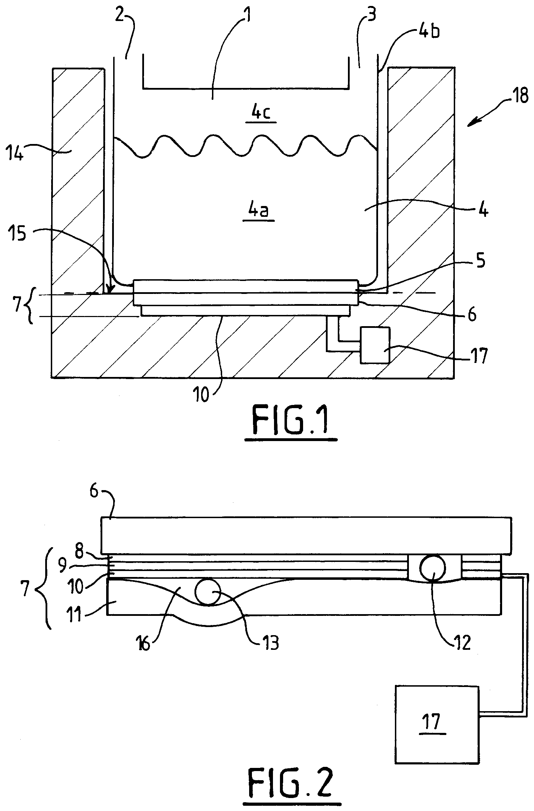

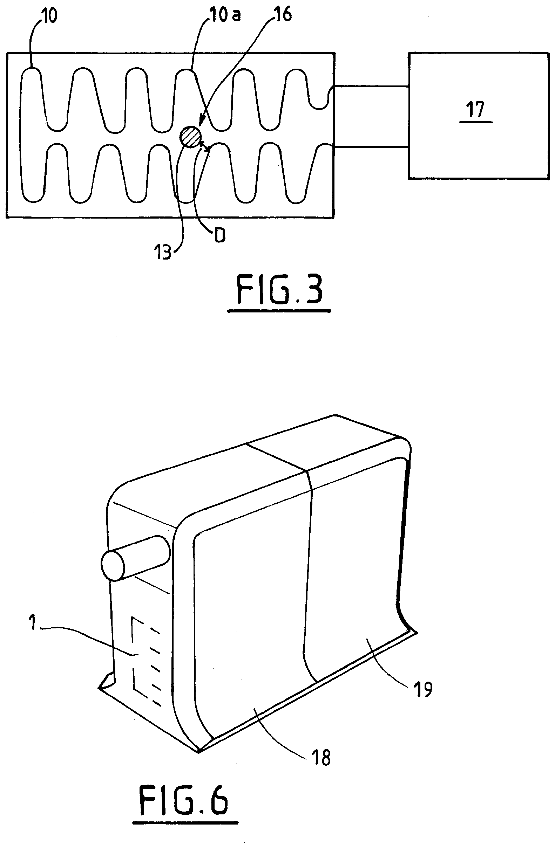

La Figure 1 représente le principe de fonctionnement d'un humidificateur 18 chauffant pour appareil d'assistance respiratoire 19, c'est-à-dire ventilateur médical, selon la présente invention, comme illustré en Figure 6.

L'humidificateur 18 comprend un boitier 14 dans lequel vient se loger un réservoir 1 d'eau 4d à chauffer, comme schématisé en Figure 1.

Le réservoir 1 est typiquement composé d'un bac 4 à eau 4d comprenant un fond 4a de bac incorporant une plaque métallique 5, appelée ci-après « deuxième plaque métallique 5 », et se prolongeant par une paroi périphérique 4b solidaire du fond 4a. L'eau contenue dans le bac 4 est donc en contact avec le fond 4a, la paroi périphérique 4b et la deuxième plaque métallique 5 du réservoir 1. La deuxième plaque métallique 5 occupe préférentiellement la majorité de la surface du fond 4a du bac 4. Le bac 4 peut être en matériau plastique et la deuxième plaque métallique 5 en aluminium ou en alliage d'aluminium.

Par ailleurs, le boitier 14 de l'humidificateur 18 comprend une structure chauffante 7 agencée dans le fond 15 dudit boitier 14, qui comprend une première plaque métallique 6 et un (ou plusieurs) élément chauffant 10 agencé de manière à transmettre la chaleur produite par l'élément chauffant 10 à la première plaque métallique 6.

En fait, la deuxième plaque métallique 5 du réservoir 1 vient en contact direct avec la première plaque métallique 6 de la structure chauffante 7 du boitier 14 lorsque le réservoir lest logé dans le boitier 14 de manière à transmettre la chaleur émise par la plaque métallique 6 de la structure chauffante 7 à la deuxième plaque métallique 5 du réservoir 1, puis ensuite à l'eau 4d en contact avec la deuxième plaque métallique 5 du réservoir 1.

Le réservoir 1 comprend une entrée d'air 2 et une sortie 3 d'air de sorte que l'air puisse entrer dans le volume interne 4c du bac 4 et s'y charger en vapeur d'eau provenant de la vaporisation d'une partie de l'eau 4d contenue dans le bac 4. L'air humidifié peut alors être évacuée du bac 4 par la sortie 3 d'air, puis être ensuite dirigé vers les voies aériennes du patient.

Par ailleurs, une première sonde de température 12 est agencée de manière à être en contact direct avec la première plaque métallique 6 et ainsi pouvoir mesurer la température de la première plaque métallique 6 de la structure chauffante 7, et le boitier 14 de l'humidificateur comprend en outre une seconde sonde de température 13 qui vient en contact ou quasi-contact de l'élément chauffant 10 tout en étant isolée thermiquement de la première plaque métallique 6.

La Figure 2 représente une vue en coupe transversale d'un mode de réalisation de la structure chauffante 7 de l'humidificateur 18 de la Figure 1. On voit que cette structure chauffante 7 comprend la première plaque métallique 6, par exemple en aluminium ou en alliage d'aluminium, une couche adhésive 8, une première couche isolante électriquement 9 assurant une isolation électrique, l'élément chauffant 10, telle une piste résistive 10 qui dissipe de la chaleur lorsqu'elle est parcourue par un courant électrique, et une seconde couche isolante électriquement et thermiquement 11.

La couche adhésive 8 est prise en sandwich entre la première plaque métallique 6 et la première couche isolante électriquement 9, alors que la couche isolante électriquement 9 est prise en sandwich entre la couche adhésive 8 et la piste résistive 10, et que la piste résistive 10 est prise en sandwich entre la première couche isolante électriquement 9 et la seconde couche isolante électriquement et thermiquement 11.

La première sonde de température 12 est agencée de sorte d'être en contact direct avec la première plaque métallique 6, alors que la seconde sonde de température 13 est agencée de sorte d'être en contact avec une zone proche de la piste résistive 10 formant l'élément chauffant 10, tout en étant isolée thermiquement de la première plaque métallique 6. Ceci est mieux visible sur la Figure 3 qui représente une vue schématique de dessus de la structure chauffante 7 de la Figure 2.

La seconde couche isolante électriquement et thermiquement 11 assure une isolation électrique et thermique de manière notamment à diriger le flux thermique, c'est-à-dire la chaleur, issu de l'élément chauffant 10 en direction de la plaque métallique 6.

La première couche isolante électriquement 9 et/ou la seconde couche isolante électriquement et thermiquement 11 sont formées de silicone, de polyimide, tel un film polyimide, ou analogue.

Par ailleurs, la couche adhésive 8 peut être formée de colle. Elle sert à assurer une adhésion de la couche isolante électriquement 9 à la plaque métallique 6.

Une telle architecture avec positionnement des première et seconde sondes de température 12, 13 selon la présente invention permet d'améliorer le pilotage du chauffage de l'humidificateur 18 en assurant les besoins de performance et de sécurité susmentionnés.

En particulier, cette disposition particulière des première et seconde sondes de température 12, 13 permet d'obtenir deux mesures différentes de température, dont la différence évolue en fonction de ce qui se trouve de l'autre côté de la plaque chauffante, c'est-à-dire de la quantité d'eau résiduelle dans le bac 4. Cette variabilité de la différence de température entre les mesures opérées par les deux sondes 12, 13 peut être utilisée pour estimer, par exemple à l'aide d'un algorithme approprié, le niveau d'eau 4d restant dans le bac 4 du réservoir 1 et détecter quand le bac 4 du réservoir 1 est vide pour par exemple interrompre la chauffe. Ceci permet alors de limiter les consommations électriques inutiles tout en réduisant le risque de brûlure pour le patient par une plaque trop chaude et/ou un gaz trop chaud.

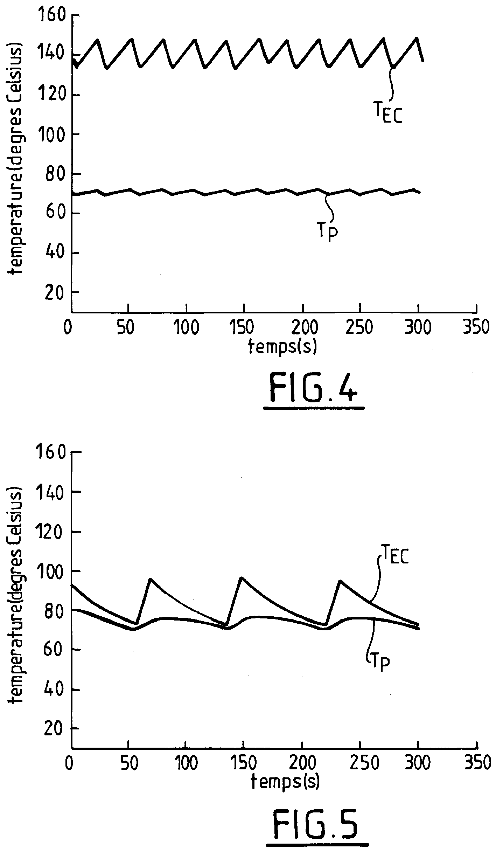

La Figure 4 représente l'évolution au fil du temps (en secondes : « s ») de la mesure de température (Tp) de la plaque 6, donnée par la sonde de température 12 en contact avec la plaque métallique 6, et l'évolution de la mesure de température (T_{EC}) de l'élément chauffant 10, donnée par la sonde 13 isolée thermiquement de la plaque métallique 6, dans le cas où le bac 4 du réservoir 3 est rempli d'un volume d'eau 4c, alors que la Figure 5 représente les mêmes évolutions de température dans le cas où le bac 4 du réservoir 3 est vide. Les températures sont exprimées en °C.

Une comparaison de ces Figures 4 et 5 permet de constater la différence d'écart entre les températures mesurées (Tp, T_{EC}) par les deux sondes de température 12, 13 selon que le bac 4 est plein ou vide d'eau. Ainsi, lorsque le bac 4 est rempli d'eau 4d, cet écart de température est important. Ceci s'explique par le fait que la chaleur générée par l'élément chauffant 10 est conduite par les plaques métalliques 5, 6 pour être transmise au volume d'eau 4d. Ce volume d'eau joue alors le rôle de capacité thermique et la température (Tp) mesurée au niveau de la plaque métallique 6 par la première sonde 12 est diminuée en conséquence. En revanche, lorsque le bac 4 du réservoir 1 est vide (i.e. absence d'eau ; présence d'air), la chaleur générée par l'élément chauffant 10 ne peut être transmise qu'à l'air contenu dans le bac 4 du réservoir 1. L'air conduisant beaucoup moins bien la chaleur que l'eau, la quantité de chaleur reste importante au niveau de la plaque métallique 6.

Dans les deux cas, le pilotage de l'élément chauffant 10 par des moyens de pilotage qui recueillent et traitent les signaux de mesure des sondes 12, 13 permet de maintenir une température constante au niveau de la première plaque métallique 6 par le biais d'un asservissement basé sur la mesure de température délivrée par la première sonde 12. Cependant, pour maintenir cette température lorsque le réservoir 1 est rempli d'eau 4d, il faut générer plus de chaleur que s'il était vide et donc élever davantage la température de l'élément chauffant 10 mesurée par la deuxième sonde de température 13.

Selon la présente invention, on utilise cette propriété pour détecter l'absence d'eau 4d dans le réservoir 1, en comparant l'écart entre les températures mesurées par les première et seconde sondes 12, 13 à un seuil de référence, par exemple un seuil préenregistré accessible au logiciel de pilotage de l'élément chauffant 10.

Quand l'écart calculé passe en-dessous de ce seuil de référence, l'élément chauffant 10 cesse d'être alimenté en courant électrique, ce qui permet, d'une part, de réduire la consommation de courant lorsqu'elle n'est pas utile et, d'autre part, de limiter la chauffe des première et première plaques métalliques 6, 5 lorsque la chaleur ne peut pas être dissipée dans l'eau 4d, de manière à éviter ou minimiser les risques de brûlure lorsque le patient manipule l'humidificateur.

De plus, ceci permet également de ne pas chauffer directement l'air contenu dans le bac 4 sans ajout d'humidité, ce qui pourrait également conduire à un risque de brûlure du patient du fait d'un gaz trop chaud.

L'humidificateur 18 de l'invention peut être logé dans ou en communication fluidique avec un appareil d'assistance respiratoire 19 adapté pour recevoir un tel humidificateur 18, comme illustré sur le mode de réalisation de la Figure 6. Celui-ci représente un mode de réalisation dans lequel un humidificateur 18 selon l'invention est raccordé fluidiquement à la sortie de gaz d'un ventilateur 19 médical de manière à humidifier le gaz, tel de l'air, délivré par ledit ventilateur 19 et à obtenir ainsi de l'air humidifié pouvant être administré ensuite aux voies aériennes d'un individu, typiquement d'un patient souffrant d'une maladie ou insuffisance respiratoire, au moyen d'une interface patient adaptée, tel un masque respiratoire, des canules nasales ou similaire, alimentée en gaz humidifié via un conduit de gaz, tel un tuyau souple.

En particulier, l'ensemble de ventilation ainsi formé, qui comprend l'humidificateur 18 selon l'invention et un ventilateur 19 médical, c'est-à-dire un appareil d'assistance respiratoire, est bien adapté au traitement des patients à domicile, notamment de patient souffrant d'une maladie respiratoire de type apnée du sommeil, syndrome d'obésité hypoventilation, broncho-pneumopathie chronique obstructive (BPCO) ou syndrome de détresse respiratoire aiguë (SDRA).

## Revendications

1. Humidificateur (18) pour appareil d'assistance respiratoire (19) comprenant un boitier (14) conçu pour loger un réservoir (1) d'eau à chauffer, ledit boitier (14) comprenant :
- une structure chauffante (7) agencée dans le fond (15) dudit boitier (14), ladite structure chauffante (7) comprenant une première plaque métallique (6) et au moins un élément chauffant (10) agencé de manière à transmettre la chaleur produite par ledit au moins un élément chauffant (10) à ladite première plaque métallique (6), et
- une première sonde de température (12) en contact direct avec la première plaque métallique (6),
**caractérisé en ce que** le boitier (14) comprend en outre une seconde sonde de température (13) en contact ou quasi-contact de l'élément chauffant (10) et isolée thermiquement de la première plaque métallique (6).

2. Humidificateur selon la revendication précédente, **caractérisé en ce que** la seconde sonde de température (13) est en quasi-contact de l'élément chauffant (10) en étant en contact avec une zone (16) située autour et à proximité immédiate de l'élément chauffant (10).

3. Humidificateur selon l'une des revendications précédentes, **caractérisé en ce que** l'élément chauffant (10) comprend au moins un élément résistif (10a) conçu pour dissiper de la chaleur lorsqu'il est parcouru par un courant électrique.

4. Humidificateur selon l'une des revendications précédentes, **caractérisé en ce que** l'élément chauffant (10) comprend au moins un élément résistif (10a) de forme longiligne, notamment filaire.

5. Humidificateur selon l'une des revendications précédentes, **caractérisé en ce que** l'élément chauffant (10) est relié électriquement à une source de courant électrique (17).

6. Humidificateur selon l'une des revendications précédentes, **caractérisé en ce qu'**au moins une couche adhésive (8) permet de solidariser l'élément chauffant (10) à la première plaque métallique (6) de la structure chauffante (7).

7. Humidificateur selon la revendication 6, **caractérisé en ce que** ladite au moins une couche adhésive (8) est prise en sandwich entre la première plaque métallique (6) et une première couche isolante électriquement (9).

8. Humidificateur selon l'une des revendications 6 et 7, lorsqu'elles dépendent de la revendication 3 ou 4, **caractérisé en ce que** au moins une première couche isolante électriquement (9) est prise en sandwich entre ladite au moins une couche adhésive (8) et l'élément résistif (10a).

9. Humidificateur selon la revendication 3 ou 4, **caractérisé en ce que** l'élément résistif (10a) est pris en sandwich entre au moins une première couche isolante électriquement (9) et au moins une seconde couche isolante électriquement et thermiquement (11).

10. Humidificateur selon la revendication 9, **caractérisé en ce que** ladite au moins une première couche isolante électriquement (9) et/ou ladite au moins une seconde couche isolante électriquement et thermiquement (11) sont formées de silicone ou de polyimide, tel un film en polyimide.

11. Humidificateur selon l'une des revendications précédentes, **caractérisé en ce que** les première et seconde sondes de température (12, 13) sont reliées électriquement à des moyens de pilotage, de préférence les moyens de pilotage comprennent une carte électronique.

12. Humidificateur selon l'une des revendications précédentes, **caractérisé en ce qu'**il comporte un réservoir (1) à eau formé d'un bac (4) comprenant un fond (4a) de bac incorporant une deuxième plaque métallique (5), ladite deuxième plaque métallique (5) du réservoir (1) venant en contact direct avec la première plaque métallique (6) de la structure chauffante (7) du boitier (14) lorsque le réservoir (1) est logé dans le boitier (14).

13. Appareil d'assistance respiratoire (19), tel un ventilateur médical, comprenant un humidificateur (18) selon l'une des revendications précédentes.

14. Ensemble de ventilation (18, 19) comprenant un humidificateur (18) selon l'une des revendications 1 à 12 raccordé fluidiquement à la sortie de gaz d'un appareil d'assistance respiratoire (19), tel un ventilateur médical.

15. Ensemble de ventilation (18, 19) selon la revendication 14, **caractérisé en ce que** l'humidificateur (18) est raccordé fluidiquement à une interface patient via un conduit de gaz.

## Patentansprüche

1. Befeuchter (18) für eine Beatmungsunterstützungsvorrichtung (19), umfassend ein Gehäuse (14), das dazu konzipiert ist, einen Behälter (1) für zu aufzuheizendes Wasser aufzunehmen, wobei das Gehäuse (14) Folgendes umfasst:
- eine Aufheizstruktur (7), die im Boden (15) des Gehäuses (14) angeordnet ist, wobei die Aufheizstruktur (7) eine erste Metallplatte (6) und mindestens ein Aufheizelement (10) umfasst, das so angeordnet ist, dass es die von dem mindestens einen Aufheizelement (10) erzeugte Wärme zur ersten Metallplatte (6) überträgt, und
- einen ersten Temperaturfühler (12) in direktem Kontakt mit der ersten Metallplatte (6),
**dadurch gekennzeichnet, dass** das Gehäuse (14) weiter einen zweiten Temperaturfühler (13) in Kontakt oder Quasikontakt mit dem Aufheizelement (10) und von der ersten Metallplatte (6) thermisch isoliert umfasst.

2. Befeuchter nach dem vorstehenden Anspruch, **dadurch gekennzeichnet, dass** der zweite Temperaturfühler (13) sich in Quasikontakt mit dem Aufheizelement (10) befindet, indem er sich mit einer Zone (16) in Kontakt befindet, die sich rund um das Aufheizelement (10) und in dessen unmittelbarer Nähe befindet.

3. Befeuchter nach einem der vorstehenden Ansprüche, **dadurch gekennzeichnet, dass** das Aufheizelement (10) mindestens ein Widerstandselement (10a) umfasst, das dazu konzipiert ist, Wärme abzugeben, wenn es von einem elektrischen Strom durchflossen wird.

4. Befeuchter nach einem der vorstehenden Ansprüche, **dadurch gekennzeichnet, dass** das Aufheizelement (10) mindestens ein Widerstandselement (10a) mit einer länglichen, insbesondere drahtförmigen Form umfasst.

5. Befeuchter nach einem der vorstehenden Ansprüche, **dadurch gekennzeichnet, dass** das Aufheizelement (10) mit einer elektrischen Stromquelle (17) elektrisch verbunden ist.

6. Befeuchter nach einem der vorstehenden Ansprüche, **dadurch gekennzeichnet, dass** mindestens eine Klebstoffschicht (8) ein festes Verbinden des Aufheizelements (10) an der ersten Metallplatte (6) der Aufheizstruktur (7) ermöglicht.

7. Befeuchter nach Anspruch 6, **dadurch gekennzeichnet, dass** die mindestens eine Klebstoffschicht (8) zwischen der ersten Metallplatte (6) und einer ersten elektrisch isolierenden Schicht (9) sandwichartig eingeschlossen ist.

8. Befeuchter nach einem der Ansprüche 6 und 7, wenn von Anspruch 3 oder 4 abhängig, **dadurch gekennzeichnet, dass** mindestens eine erste elektrisch isolierende Schicht (9) zwischen der mindestens einen Klebstoffschicht (8) und dem Widerstandselement (10a) sandwichartig eingeschlossen ist.

9. Befeuchter nach Anspruch 3 oder 4, **dadurch gekennzeichnet, dass** das Widerstandselement (10a) zwischen mindestens einer ersten elektrisch isolierenden Schicht (9) und mindestens einer zweiten elektrisch und thermisch isolierenden Schicht (11) sandwichartig eingeschlossen ist.

10. Befeuchter nach Anspruch 9, **dadurch gekennzeichnet, dass** die mindestens eine erste elektrisch isolierende Schicht (9) und/oder die mindestens eine zweite elektrisch und thermisch isolierende Schicht (11) aus Silicon oder Polyimid, wie einem Polyimidfilm, gebildet sind.

11. Befeuchter nach einem der vorstehenden Ansprüche, **dadurch gekennzeichnet, dass** der erste und zweite Temperaturfühler (12, 13) mit Steuermitteln, vorzugsweise eine Platine umfassenden Steuermitteln, elektrisch verbunden sind.

12. Befeuchter nach einem der vorstehenden Ansprüche, **dadurch gekennzeichnet, dass** er einen aus einer Schale (4) gebildeten Wasserbehälter (1) umfasst, die einen Schalenboden (4a) umfasst, der eine zweite Metallplatte (5) enthält, wobei die zweite Metallplatte (5) des Behälters (1) mit der ersten Metallplatte (6) der Aufheizstruktur (7) des Gehäuses (14) in direkten Kontakt kommt, wenn der Behälter (1) im Gehäuse (14) aufgenommen ist.

13. Beatmungsunterstützungsvorrichtung (19), wie ein medizinisches Beatmungsgerät, umfassend einen Befeuchter (18) nach einem der vorstehenden Ansprüche.

14. Beatmungsanordnung (18, 19), umfassend einen Befeuchter (18) nach einem der Ansprüche 1 bis 12, der mit dem Gasauslass einer Beatmungsunterstützungsvorrichtung (19), wie eines medizinischen Beatmungsgeräts, in Fluidverbindung steht.

15. Beatmungsanordnung (18, 19) nach Anspruch 14, **dadurch gekennzeichnet, dass** der Befeuchter (18) über eine Gasleitung mit einer Patientenschnittstelle in Fluidverbindung steht.

## Claims

1. Humidifier (18) for breathing assistance apparatus (19) comprising a casing (14) designed to house a tank (1) of water to be heated, said casing (14) comprising:
- a heating structure (7) arranged in the bottom (15) of said casing (14), said heating structure (7) comprising a first metal plate (6) and at least one heating element (10) arranged so as to transmit the heat produced by said at least one heating element (10) to said first metal plate (6), and
- a first temperature sensor (12) in direct contact with the first metal plate (6),
**characterised in that** the casing (14) further comprises a second temperature sensor (13) in contact with or practically in contact with the heating element (10) and thermally insulated from the first metal plate (6).

2. Humidifier according to the preceding claim, **characterised in that** the second temperature sensor (13) is practically in contact with the heating element (10) by being in contact with a zone (16) located around and in the immediate proximity of the heating element (10).

3. Humidifier according to one of the preceding claims, **characterised in that** the heating element (10) comprises at least one resistive element (10a) designed to dissipate heat when an electric current flows through it.

4. Humidifier according to one of the preceding claims, **characterised in that** the heating element (10) comprises at least one resistive element (10a) with a slender shape, in particular like a wire.

5. Humidifier according to one of the preceding claims, **characterised in that** the heating element (10) is electrically connected to a source of electric current (17).

6. Humidifier according to one of the preceding claims, **characterised in that** at least one adhesive layer (8) makes it possible to secure the heating element (10) to the first metal plate (6) of the heating structure (7).

7. Humidifier according to claim 6, **characterised in that** said at least one adhesive layer (8) is sandwiched between the first metal plate (6) and a first electrically insulating layer (9).

8. Humidifier according to one of claims 6 and 7, when they depend on claim 3 or 4, **characterised in that** at least one first electrically insulating layer (9) is sandwiched between said at least one adhesive layer (8) and the resistive element (10a).

9. Humidifier according to claim 3 or 4, **characterised in that** the resistive element (10a) is sandwiched between at least one first electrically insulating layer (9) and at least one second electrically and thermally insulating layer (11).

10. Humidifier according to claim 9, **characterised in that** said at least one first electrically insulating layer (9) and/or said at least one second electrically and thermally insulating layer (11) are formed from silicone or polyimide, such as a polyimide film.

11. Humidifier according to one of the preceding claims, **characterised in that** the first and second temperature sensors (12, 13) are electrically connected to control means, preferably the control means comprise an electronic board.

12. Humidifier according to one of the preceding claims, **characterised in that** it comprises a tank (1) for water formed from a receptacle (4) comprising a tank bottom (4a) incorporating a second metal plate (5), said second metal plate (5) of the tank (1) coming in direct contact with the first metal plate (6) of the heating structure (7) of the casing (14) when the tank (1) is housed in the casing (14).

13. Breathing assistance apparatus (19), such as a medical ventilator, comprising a humidifier (18) according to one of the preceding claims.

14. Ventilation assembly (18, 19) comprising a humidifier (18) according to one of claims 1 to 12, fluidically connected to the gas outlet of a breathing assistance apparatus (19), such as a medical ventilator.

15. Ventilation assembly (18, 19) according to claim 14, **characterised in that** the humidifier (18) is fluidically connected to a patient interface via a gas duct.
